# EUROPEAN PATENT APPLICATION

(11) **EP 2 559 424 A1**
(43) Date of publication of application: **20.02.2013**
(21) Application number: 12177356.8
(22) Date of filing: 05.10.2006
(51) Int. Cl.: A61K 8/34, A61K 8/37, A61Q 5/00, A61Q 5/02, A61Q 9/02, A61Q 11/00, A61Q 15/00, A61Q 19/00, A61Q 19/10, A61K 8/42

(54) **Cooling composition comprising trimethyl isopropyl butanamide**

(30) Priority: 05.10.2005 US 723698 P
(62) Divisional of application: 06825560.3
(71) Applicant: Kraft Foods Global Brands LLC, Northfield, IL 60093 (US)
(72) Inventor: Kiefer, Jesse, Columbia, NJ New Jersey 07832 (US); Harvey, Joan, E., Morgantown, PA Pennsylvania 19543 (US)
(74) Representative: Wilson Gunn

(57) **Abstract**

This description provides cooling compositions that deliver a prolonged physiological cooling sensation to the skin or a mucous membrane. The cooling compositions may be present alone or in product such as a chewing gum or a confection. The cooling compositions comprise a menthyl ester and a second cooling agent, which may be WS-3 or WS-23 and optionally menthol. The menthyl ester may be a menthyl glutarate or a menthyl succinate ester, and it may be present in an amount of at least 5 weight percentage, at least 10 weight percentage, at least 20 weight percentage, at least 30 weight percentage, or at least 40 weight percentage. In many instances, the menthyl ester is present in an amount of 5 to 60 weight percentage or especially 15 to 50 wt %.

## Description

### CROSS-REFERENCE TO RELATED APPLICATIONS

This application claims the benefit of U.S. Provisional Application No. 60/723,698, filed October 5, 2005, the contents of which are incorporated herein by reference.

### FIELD

This description relates to cooling compositions that may be delivered orally or to the skin or mucous membranes. The compositions contain one or more cooling agents in combination with a menthyl ester. The cooling agent(s) and menthyl ester may be provided in one composition or they may be provided separately in distinct formulations separated over time or geographically.

### BACKGROUND

Many substances are known to provide a sensation of cooling on application and are called "cooling agents." Exemplary cooling agents include menthol, isopulegol, 3-(1-menthoxy)propane-1,2-diol, 3-(1-menthoxy)-2-methylpropane-1,2-diol, p-menthane-2,3-diol, p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, menthone, menthone glycerol ketal, menthyl lactate, 3-(1-menthoxy)ethan-1-ol, 3-(1-menthoxy)propan-1-ol, 3-(1-menthoxy)butan-1-ol, 1-melithylacetic acid N-ethylamide, 1-menthyl-4-hydroxypentanoate, 1-menthyl-3-hydroxybutyrate, N,2,3-trimethyl-2-(1-methylethyl)-butanamide, n-ethyl-t-2-c-6 nonadienamide, N,N-dimethyl menthyl succinamide, and menthyl pyrrolidone carboxylate.

Despite the existence in the prior art and in commerce of such a vast number and variety of cooling agents, a need exists for a cooling composition that will contribute a long-lasting cooling sensation to products in which it is found without unwanted harshness or flavor characteristics. It would also be desirable to provide a clean, high-quality flavor chewing gum with a good cooling effect without leaving a significant aftertaste. Including mono menthyl ester-based compounds in food, cosmetic and fragrance applications may supply a need for cooler compounds which provide strong and substantive refreshing and cooling attributes in the absence of negative taste, negative aroma and negative cooling attributes. There remains a need to provide cooling compositions which provide good onset of effect and prolonged cooling.

### SUMMARY

The compounds and compositions described herein provide cooling compositions that deliver a prolonged physiological cooling sensation to the skin or a mucous membrane. The cooling compositions may be present alone or in product such as a chewing gum or a confection.

In some instances, the cooling compositions may provide for delivering a sensation substantially similar to that delivered by WS-3 or WS-23 alone by administering a composition containing a menthyl ester but where the amount of WS-3 or WS-23 present in the composition can be reduced relative to the amount of WS-3 or WS-23 alone required to provide a substantially similar sensation.

In a first embodiment, a cooling composition may include a menthyl ester and a second cooling agent. Optionally, the composition may further contain menthol. In some instances, the second cooling agent can be WS-3 or WS-23, which may be used interchangeably. The menthyl ester may be, for instance, a menthyl glutarate ester or a menthyl succinate ester. The menthyl ester may be present in an amount of at least 5 wt. %, at least 10% wt. %, at least 20 wt. %, at least 30 wt. %, or at least 40 wt. %. In many instances, the menthyl ester can be present in an amount of 5 to 60 wt. % or especially 15 to 50 wt. %.

In some embodiments, the cooling compositions may allow the amount of the second cooling agent, such as WS-3 or WS-23 to be reduced while providing substantially the same physiological sensation. The cooling compositions therefore allow reducing or eliminating any undesired side effects or sensations associated with the second cooling agent.

In a second embodiment, a chewing gum may include a menthyl ester and a second cooling agent. Optionally, the chewing gum may further contain menthol. In some instances, the second cooling agent can be WS-3 or WS-23. The menthyl ester may be, for instance, a menthyl glutarate ester or a menthyl succinate ester. The menthyl ester may be present in an amount of at least 5 wt. %, at least 10 wt. %, at least 20 wt. %, at least 30 wt. %, or at least 40 wt. % of the cooling composition. In many instances, the menthyl ester can be present in an amount of 5 to 60 wt. % or especially 15 to 50 wt. % or 20 to 40 wt. % of the cooling composition.

In a third embodiment, a confectionary may include a menthyl ester and a second cooling agent. Optionally, the confectionary may further contain menthol. In some instances, the second cooling agent can be WS-3 or WS-23. The menthyl ester may be, for instance, a menthyl glutarate ester or a menthyl succinate ester. The menthyl ester may be present in an amount of at least 5 wt. %, at least 10 wt. %, at least 20 wt. %, at least 30 wt. %, or at least 40 wt. % of the cooling composition. In many instances, the menthyl ester can be present in an amount of 5 to 60 wt. % or especially 15 to 50 wt. % of the cooling composition. Some cooling agents useful for a confectionary include, for instance, WS-3, WS-23, WS-30, WS-14, Eucalyptus extract (p-Mehtha-3,8-Diol), Menthol (its natural or synthetic derivatives), 3,3,5-Trimethyl Cyclohexanol, Ethyl p-menthane carboxamide, N,2,3-trimethyl-2-isopropyl butanamide, Menthyl glutarate FEMA 4006, Menthyl succinate, Menthol PG carbonate, Menthol EG carbonate, Menthyl lactate, Menthone glyceryl ketal, Menthol glyceryl ether, N-tertbutyl-p-menthane-3-carboxamide, P-menthane-3-carboxylic acid glycerol ester, Methyl-2-isopryl-bicyclo (2.2.1), Heptane-2-carboxamide; and Menthol methyl ether and combinations thereof.

In a fourth embodiment, is described a method for delivering a prolonged physiological cooling sensation to the skin or a mucous membrane comprising administering a cooling composition comprising a menthyl ester and a second cooling agent. Optionally, the cooling composition may further contain menthol. In some instances, the second cooling agent can be WS-3 or WS-23. The menthyl ester may be, for instance, a menthyl glutarate ester or a menthyl succinate ester. The menthyl ester may be present in an amount of at least 5 wt. %, at least 10 wt. %, at least 20 wt. %, at least 30 wt. %, or at least 40 wt. % of the cooling composition. In many instances, the menthyl ester can be present in an amount of 5 to 60 wt. % or especially 15 to 50 wt. % of the cooling composition.

In a fifth embodiment is described a method for delivering a sensation substantially similar to that delivered by WS-3 alone comprising administering a cooling composition comprising a menthyl ester and WS-3. Optionally, the composition may further contain menthol. The menthyl ester may be, for instance, a menthyl glutarate ester or a menthyl succinate ester. The menthyl ester may be present in an amount of at least 5 wt. %, at least 10 wt. %, at least 20 wt. %, at least 30 wt. %, or at least 40 wt. % of the cooling composition. In many instances, the menthyl ester can be present in an amount of 5 to 60 wt. % or especially 15 to 50 wt. % of the cooling composition.

In a sixth embodiment is described a method for delivering a sensation substantially similar to that delivered by WS-23 alone comprising administering a cooling composition comprising a menthyl ester and WS-23. Optionally, the cooling composition may further contain menthol. The menthyl ester may be, for instance, a menthyl glutarate ester or a menthyl succinate ester. The menthyl ester may be present in an amount of at least 5 wt. %, at least 10 wt. %, at least 20 wt. %, at least 30 wt. %, or at least 40 wt. % of the cooling composition. In many instances, the menthyl ester can be present in an amount of 5 to 60 wt. % or especially 15 to 50 wt. % of the cooling composition.

### DETAILED DESCRIPTION

The description extends to products that otherwise may contain cooling compositions such as preferably flavoring agents, foodstuffs, confections, beverages, gums, dentifrices, mouthwashes, toiletries, liniments lotions for topical application and cigarettes, such products comprising a composition that provides a physiological sensation substantially the same as that provided by menthol. The cooling compositions provide a substantially cooling physiological sensation.

Menthol is known for its physiological cooling effect on the skin and mucous membranes of the mouth and has been extensively used as a flavouring agent, being a major constituent of oil of peppermint in foodstuffs, beverages, dentifrices, mouthwashes, etc. and as a component in a wide range of toiletries, liniments and lotions for topical application. Menthol is also a well known tobacco additive for producing a "cool" sensation in the mouth when smoking. Carvomenthol has also been reported as having a physiological cooling effect as have N,N-dimethyl-2-ethyl butanamide and N,N-diethyl-2-ethyl butanamide as described in, for instance, French Patent No. 1,572,332.

It is well established that the "cooling" effect of menthol is a physiological effect due to the direct action of menthol on the nerve endings of the human body responsible for the detection of hot and cold. Menthol directly stimulates cold receptors. Some non-menthol compounds providing a physiological effect similar to menthol are described in U.S. Patent 4,296,255, herein incorporated by reference.

Peppermint oil may be used to create a "cooling" in oral products such as toothpaste, mouthwash, chewing gum, candy and other food products. Peppermint oil generally comprises about 45-55% menthol, about 20-25% menthone, about 5% menthyl acetate, about 5% eucalyptol and many other constituents. Peppermint oil is even used in non-peppermint products, such as spearmint or wintergreen flavored products, in order to create this desired cooling effect. However, peppermint notes are then found in the resulting non-peppermint flavored products.

Menthol is also known for its physiological cooling effect on the skin and mucous membranes of the mouth. Being a major constituent of peppermint oil, menthol has been used extensively in foods, beverages, dentifrices, mouthwashes, toiletries, lotions and the like. The disadvantages of using menthol, however, are its strong minty odor and the harsh notes it imparts to compositions in which it is found. Menthol has been used in conjunction with other cooling agents because, among other things, it acts to prepare taste buds to receive non-menthol cooling agents. Menthol provides a light, fresh, minty sensation and in some regards prepares taste buds to receive a cooling sensation. It has been reported that some cooling agents, e.g. WS-3 and WS-23, may in fact deliver an initial warming sensation if supplied without menthol.

Most confectionery products which are promoted for breath freshening are mint flavored products which contain moderate to high levels of menthol. The disadvantages of using menthol, however, are its strong minty odor and the harsh notes it imparts to compositions in which it is found. A need, therefore, exists for a cooling composition that will contribute a long-lasting cooling sensation to products in which it is found without the unwanted harshness or flavor characteristics that come from adding menthol.

In some consumer products, especially chewing gums, it is desirable to provide a burst of intense flavor over a slow, gentle flavor release. In order to provide a favorable flavor impact, some chewing gum manufacturers have added flavors to the coating of a coated chewing gum. These flavors include spearmint flavor, peppermint flavor, wintergreen flavor and fruit flavors. In addition, very strong flavors such as menthol have often been used to provide a burst of flavor. However, at concentrations effective to provide a burst of flavor, menthol or mint flavors also manifest a bitter, harsh, burning taste sometimes described as a "harsh note".

Efforts have been directed at perfecting the use of physiological cooling agents within chewing gum formulations to enhance flavor composition and control their release to enhance the flavor of chewing gum. U.S. Pat. No. 5,326,574 discloses a process for codrying the physiological cooling agent 3-1-menthoxypropane-1,2-diol with a food acceptable, watersoluble carrier and mixing the resulting product into chewing gum.

L-Monomenthyl glutarate has the chemical name (L)-Monomenthane-3-yl glutarate, is sometimes known as Pentadienoic acid, mono[5 methyl-2-1(1-methylethyl) cyclohexyl]ester, [1L]; [1R(-)] monomenthyl glutarate, and has the chemical formula C₁₅H₂₆O₄. It may be located by JECFA Number 1414, FEMA number 4006 and CAS number 220621-22-7. It is present as a clear viscous fluid having a minty, menthol-like aroma.

### Cooling agents

The term "physiological cooling agent" encompasses any number of physiological cooling agents. However, in the context of this description, the term "physiological cooling agent" does not include traditional flavor-derivatives such as menthol or menthone. Preferred physiological cooling agents do not have a perceptible flavor of their own, but simply provide a cooling effect.

An optional additional component of the cooling compositions is a physiological cooling agent. Suitable levels of the cooling agent are from about 0.001 to about 85%, preferably from about 0.01 to about 50%, more preferably from about 0.05 to about 15% by weight of the cooling composition, and still more preferably from about 0.10 to about 5% by weight of the composition.

Generally, the compositions will contain an amount of the active cooling compounds sufficient to stimulate the cold receptors in the areas of the skin or mucous membrane with which the compositions come into contact and thereby promote the desired cold sensation. As the degree and longevity of cooling sensation varies from compound to compound the quantity of stimulant used in each composition will vary widely. As a guide, it may be said that, with the more active compounds, a significant cooling sensation, which, in some cases, may persist for several hours, can be achieved upon application to the mucosa or skin of as little as 0.05 ml. of a 1.0% weight percent solution of the active ingredient in ethanol. For the less active compounds a significant cooling effect can be achieved only with more concentrated solutions, e.g. 5.0% by weight or more of the active ingredient. It must also be admitted that such skin tests are somewhat subjective, some individuals experiencing a greater or lesser cooling sensation than others when subjected to the same test.

Cooling agents are well known in the art and are described in, for instance, U.S. Patents 4,032,661, 4,070,449, 4,033,994, 4,296,093, 4,296,255, 4,230,688, 4,034,109, 4,020,153, 4,136,163, 5,266,592, U.S. Publication Nos. 2004/0067970 and 2005/0019455, and by Leffingwell et al.*,* "Cool without Menthol & Cooler than Menthol and Cooling Compounds as Insect Repellents," the disclosures of which are herein incorporated by reference. A test for physiological cooling agents is described in GB-A-1,452,291, published Oct. 13, 1976, reproduced in part herein below for convenience. Cooling agents are well known in the art. Preferred physiological cooling agents do not have a perceptible flavor of their own, but simply provide a cooling effect. Since the physiological cooling agents do not have their own perceptible flavor, they can be used with other types of flavors to offer new and unique advantages, such as breath freshening. Several U.S. and foreign references disclose specific compounds and classes of compounds that are physiological cooling agents that may be used in the present compositions. Some of these disclose the use of physiological cooling agents in chewing gum. These include, for instance, U.S. Pat. No. 5,451,404 (a ketal combined with another coolant (menthol or carboxamides)); U.S. Pat. No. 5,372,824 (physiological cooling agents and reduced menthol); U.S. Pat. No 5,348,750 (menthone ketals); U.S. Pat. No. 5,326,574 (a spray dried 3-1-menthoxypropane-1,2-diol- ); U.S. Pat. No. 5,266,592 (menthone glycerol ketals); U.S. Pat. No. 5,165,943 (a cyclodextrin complex with physiological cooling agents); U.S. Pat. No. 5,009,893 (p-menthane carboxamide physiological cooling agent with menthol for reduced bitterness); U.S. Pat. No. 4,459,425 (3-1-menthoxypropane-1,2-diol); U.S. Pat. No. 4,296,093 (substituted cyclohexanamides); U.S. Pat. Nos. 4,248,859 and 4,318,900 (alkyl substituted alicyclic carboxylic acids, esters or amides); U.S. Pat. Nos. 4,157,384 and 4,029,759 (various 3-substituted p-menthanes); U.S. Pat. No. 4,081,480 (alpha-oxy(oxo)mercaptan alkanes); U.S. Pat. No. 4,070,449 (sulphoxides and sulphones); U.S. Pat. Nos. 4,060,091; 4,190,643 and 4,136,163 (substituted p-menthane-3-carboxamides); U.S. Pat. Nos. 4,153,679; 4,296,255 and 4,230,688 (acyclic carboxamides); U.S. Pat. No. 4,034,109 (acyclic sulphonamides and sulphinamides); U.S. Pat. No. 4,033,994 (p-menthane-3-carboxylates); U.S. Pat. Nos. 3,793,446 and 3,644,613 (ketoesters of menthol); U.S. Pat. No. 3,720,762 (spilanthol with menthol or peppermint oil); Canadian Patent No. 2,101,790 (carbonic acids having free polar groups); German Patent No. 2,608,226 (menthyl lactate); German Patent No. 2,433,165 (N-acetylglycine menthyl ester); French Patent No. 2,577,922 (L-menthyl-3-hydroxybutyrate); Japanese Patent No. 94/065023 (2-isopropenyl-5-methylcyclohexanol); Great Britain Patent No. 1,502,680 (bicyclic acids, esters, amides and substituted menthanols); Great Britain Patent No. 1,476,351 (cyclic and acyclic amides, ureas and sulphonamides); Great Britain Patent No. 1,442,998 (trialkylsubstituted cyclohexane carboxamides); Great Britain Patents Nos. 1,421,744 and 1,421,743 (novel amides); Great Britain Patent No. 1,411,786 (cyclohexanamides); Great Britain Patent No. 1,404,596 (acyclic secondary and tertiary alkanols); PCT Publication No. WO 97107771 (menthyl succinate and carboxamides); PCT Publication No. WO 96/28133 (coolant composition for comestibles); PCT Publication No. WO 96/17524 (a cooling composition comprising N-substituted p-menthane carboxamides and menthol); PCT Publication No. WO 94/010117 (cyclohexanol derivatives); and U.S. S. Pat. No. 3,639,569 (physiological cooling agents). U.S. Patents 4,032,661, 4,070,449, 4,033,994, 4,296,093, 4,296,093, 4,296,255, 4,230,688, 4,034,109, 4,020,153, 5,009,893, 5,698,181, 5,266,592, U.S. Publication Nps. 2004/0067970 and 2005/0019455, and Leffingwell *et al.,* "Cool without Menthol & Cooler than Menthol and Cooling Compounds as Insect Repellents," the disclosures of which are also herein incorporated by reference provide yet further examples. A test for physiological cooling agents is described in GB-A-1,452,291, published Oct. 13, 1976, reproduced in part herein below for convenience.

Particular examples of physiological cooling agents include, for instance, substituted p-menthanes, substituted p-menthane-carboxamides (e.g., N-ethyl-p-menthane-3-carboxamide (FEMA 3455)), acyclic carboxamides, substituted cyclohexanamides, substituted cyclohexane carboxamides, substituted ureas and sulphonamides, and substituted menthanols (all from Wilkinson Sword); hydroxymethyl and hydroxyethyl derivatives of p-menthane (from Lever Bros.); menthyl succinate; 2-mercapto-cyclo-decanone (from International Flavors and Fragrances); 2-isopropanyl-5-methylcyclohexanol (from Hisamitsu Pharmaceuticals, hereinafter "isopregol"); hydroxycarboxylic acids with 2-6 carbon atoms; menthone glycerol ketals (FEMA 3807, trade name FRESCOLAT® type MGA); 3-1-menthoxypropane-1,2-diol (from Takasago, FEMA 3784, (hereinafter "TCA")); and menthyl lactate; (from Haarman & Reimer, FEMA 3748, trade name FRESCOLAT® type ML).

While any of the above-disclosed physiological cooling agents may be used in chewing gum, some preferred physiological cooling agents are substituted p-menthane carboxamides (PMC), such as those disclosed in U.S. Pat. Nos. 4,060,091; 4,190,643 and 4,136,163, all assigned to Wilkinson Sword, especially N-ethyl-p-menthane-3-carboxamide (called WS-3); acyclic carboxamides (AC), such as those disclosed in U.S. Pat. Nos. 4,296,255; 4,230,688; and 4,153,679; all assigned to Wilkinson Sword, especially N-2,3-trimethyl-2-isopropyl butanamide (called WS-23); menthone glycerol ketal (MGK); menthyl lactate (ML); menthyl succinate (MS); and 3-1-menthoxypropane-1,2-diol (TCA).

Other preferred physiological cooling agents are described in W097/06695. Some include menthol, peppermint oil, N-substituted-p-menthane-3-carboxamides, acyclic tertiary and secondary carboxamides, 3-1-menthoxy propan-1,2-diol and mixtures thereof. Some carboxamides found most useful are those described in U.S. Pat. No. 4,136,163, Jan. 23, 1979 to Watson et al., and U.S. Pat. No. 4,230, 688, Oct. 28, 1980 to Rowsell et al. The carboxamides in U.S. Pat. No. 4,136,163 are N-substituted-p-menthane-3-carboxamides. N-ethyl-p-menthane-3-carboxamide, commercially available as WS-3 from Wilkinson Sword, is preferred herein. The carboxamides of U.S. Pat. No. 4,230,688 are certain acyclic tertiary and secondary carboxamides, of which trimethyl isopropyl butanamide, commercially available as WS-23 from Wilkinson Sword is one preferred cooling agent for use herein. Others include WS-3, WS-14, WS-23 and the like.

The following test procedure can be used as a means to identify compounds having a physiological cooling activity. This test is intended purely as a means for identifying compounds having a physiological cooling agent activity and useful in the present compositions and for giving an indication of the different relative activities of the compounds, as between themselves and as compared with menthol, when applied in particular manner to a particular part of the body. The results are not necessarily indicative of the activity of these compounds in other formulations and other parts of the body where other factors come into play. For example, a controlling factor in the onset of cooling effect, its intensity and longevity will be the rate of penetration of the compounds through the epidermis or mucous membrane and this will vary in different locations on the human body. The formulation of actual products will therefore be done largely on an empirical basis although the test results and other figures given herein will be useful as a guide, particularly in the formulation of products for oral administration, since the test procedure to be described involves oral application of the compound. A similar test may, of course, be devised for the purposes of measuring the relative activities of the compounds of another area of the body, for example, the face or forearm, and this will be a useful guide in the choice of compounds to be used in preparations for external topical usage. It will also be noted that the described test procedure can be done on a statistical basis. This is necessary since sensitivity to these compounds will vary not only from compound to compound and from one part of the body to another, but also from one individual to another. Tests of this nature are commonly used in the testing of the organoleptic properties e.g. taste and smell of organic and inorganic compounds, see Kirk-Othmer: Encyclopedia of Chemical Technology, 2nd Ed. (1967) Vol. 14, pages 336-344.

The following test procedure is aimed at determining the minimum quantity of the test compound required to produce a noticeable cooling effect in a person of average sensitivity, this minimum quantity being termed the threshold for that particular compound. The tests are carried out on a selected panel of 6 people of median sensitivity to 1-menthol.

In some embodiments, the following procedure may be used to select a panel. Known quantities of 1-menthol in solution in petroleum ether (bp. 40-60 °C.) are placed on 5 mm squares of filter paper, after which the solvent can be allowed to evaporate. A panel of observers can be enrolled and asked to place one impregnated square at a time on the tongue and to report on the presence or absence of a cooling effect. The quantity of 1-menthol on each impregnated square is gradually reduced from a value substantially above 0.25 micrograms per square to substantially below 0.25 micrograms, the precise range being immaterial. Conveniently, one starts with squares containing 2.0 micrograms being half that of the preceding square, i.e. the second test square will contain 1.0 microgram, the third 0.5 microgram, and so on. Each quantity can be tested on the tongue at least ten times. In this way, the thresholds to cold receptor stimulus by 1-menthol are determined for each individual of the panel, the threshold for each individual being that amount of 1-menthol for which, in a series of not less than ten test applications, a cooling effect is reported 50% of the time. Six panel members can be selected whose threshold to 1-menthol is in the range 0.1 micrograms to 10 micrograms and whose average threshold is approximately 0.25 micrograms, this select panel being regarded as the test panel of average sensitivity.

To test the activity of cooling agents, the above procedure can be repeated using only the six selected panel members of average sensitivity to 1-menthol. The individual thresholds for each test compound on each of the six selected panel members are determined and averaged. Those compounds whose average threshold on the select test panel is 100 micrograms or less, preferably 50 micrograms or less are regarded as having cooling activity.

The balance of the cooling composition can be made up of a suitable appropriate carrier, such as water or a bulk sweetener, described in more detail below.

### Menthyl esters

U.S. Pat. No. 3,111,127 incorporated herein by reference discloses such monomenthyl esters as monomenthyl succinate, monomenthyl αα-dimethyl succinate and monomenthyl 2-methylmaleate. U.S. Patent No. 4,150,052 incorporated herein by reference discloses the use of N-ethyl p-menthane-3-carboxamide for its physiological cooling action on the skin, and discloses its use in, for example, chewing gum. U.S. Patent Nos. 5,725,865 and 5,843,466 incorporated herein by reference disclose the use of mono menthyl succinate for its physiological cooling action and disclose its use in, for example, carbonated beverages, alcoholic beverages and chewing gum. U.S. Patent No. 6,365,215 incorporated herein by reference discloses the use of mono menthyl glutarate in oral sensory perception-affecting compositions for use with such consumable materials as chewing gums. U.S. Pat. No. 6,451,844 incorporated herein by reference discloses the use of menthyl 2-pyrrolidone-5-carboxylate (QUESTICE L, Quest International, B.V. ofNaarden, Netherlands) in skin care and hair care compositions for their insect repellency properties.

Furthermore, L-mono menthyl glutarate has been registered as a GRAS flavoring substance, FEMA No. 4006 and, in Smith et al., "GRAS Flavoring Substances 20", Food Technology, Vol. 55, No. 12, December 2001 at page 53 is indicated to have uses in nonalcoholic beverages, alcoholic beverages, chewing gum, confectionery frosting, hard candy, soft candy and snack foods.

A number of the mono menthyl half acid ester derivatives are found to be useful such as (i) L-menthyl hydrogen adipate (n=3); (ii) L-menthyl hydrogen pimelate (n=4); and (iii) L-menthyl hydrogen suberate (n=5) as disclosed by Rule et al., "Optical Activity and the Polarity of Substituent Groups Part VIII. Growing-chain Effects and the ortho-Effect in Benzoic Esters", J. Chem.Soc. 1928 (Part 1), pp. 1347-1361.

In addition, a lower adjacent methylene homologue of the genus of novel compounds is disclosed in "SciFinder" (Nov. 20, 2002; Trademark of Chemical Abstracts Services), to wit: malonamic acid, p-menth-3-yl ester, (.+-.)-(8CI) having CAS Registry Number 6129-88-0.

### Definitions

By "cooling agent" is meant any agent whether described herein, known in the art as producing, or otherwise capable of producing a sensation described as cooling by those experiencing it on the skin, oral cavity or mucous membranes.

By "aroma" is meant a minty or freshening physiological sensation perceived as a sense of smell.

By "cooling effect" is meant a sensation described as cooling by those experiencing it on the skin, oral cavity or mucous membranes.

By phrases such as "the amount of WS-23 present in the composition, chewing gum or confection is reduced relative to the amount of WS-23 alone required to provide the substantially similar sensation" or "the amount of WS-3 present in the composition, chewing gum or confection is reduced relative to the amount of WS-3 alone required to provide the substantially similar sensation" is meant that on average, respondents evaluating the "cooling" sensation provided by the cooling composition quantify the "cooling" sensation as substantially equivalent to that provided by WS-23 or WS-3 alone even though WS-23 or WS-3 are present in amounts that are at least 5%, at least 10%, at least 20%, at least 30% or at least 40% or even 50% less by mass.

By "Menthyl ester" is meant a class of compounds such as those described in, for instance, U.S. Patent 3,111,127, U.S. Patent 6,365,215 and U.S. Patent 6,884,906 the disclosures of which are herein incorporated by reference, including monomenthyl succinate, dimenthyl succinate, monomenthyl α,α-dimethyl succinate and monomenthyl 2-methylmaleatementhyl glutarate, FEMA 4006, as described, *supra.* Also, are intended derivatives thereof, such as for example, the menthyl half acid ester derivatives set forth in U.S. Patent 6,884,906. The term is also intended to embrace the alkali metal salts and alkaline earth metal salts of the menthyl compounds such as monomenthyl succinate and monomenthyl glutarate.

### Cooling Compositions

The cooling compositions can be used in any products intended for oral, skin or mucosal delivery as a component for providing sensation of cooling and for providing a physiological effect that is substantially cooling. The products for which the compositions are useful include, but are not limited to, food and drink, such as candies, drops, chewing gums, tablets, chocolates, cakes, cookies, snack food, bread, tea, coffee, juice, fruit drinks, fruit wine, dairy drinks, carbonated beverages, alcoholic beverages and seasonings; and oral care preparations, such as mouthwash, toothpaste, nebulizers, drinks, medicinal drops, gargles, and chewables.

The following illustrate a more comprehensive range of products into which the active cooling composition can be incorporated. These include, for instance, edible or potable compositions including alcoholic and non-alcoholic beverages, confectionery, chewing gum; cachous; ice cream; jellies. These further include toiletries including after shave lotions, shaving soaps, creams and foams, toilet water, deodorants and antiperspirants, "solid colognes," toilet soaps, bath oils and salts, shampoos, hair oils, talcum powders, face creams, hand creams, sunburn lotions, cleansing tissues, dentifrices, toothpicks, mouthwashes, hair tonics, eye drops. Additionally, the range extends to medicaments including antiseptic ointments, pile ointments, liniments, lotions, decongestants, counter-irritants, cough mixtures, throat lozenges, antacid and indigestion preparations, oral analgesics. Also contemplated are tobacco preparations including cigars, cigarettes, pipe tobacco, chewing tobacco and snuff; tobacco filters, especially filter tips for cigarettes. Even further are contemplated miscellaneous compositions such as water soluble adhesive compositions for envelopes, postage stamps, adhesive labels etc.

The edible and potable compositions will contain the active cooling composition in combination with an edible carrier and usually a flavoring or coloring agent. In general, the cooling composition may be present in amounts in the range 0.0005 to 5% by weight based on the total composition. Similar considerations apply to the formulation of beverages. In general, the amount of the cooling composition used will generally be in the range 0.0005 to 2.5% by weight based on the total composition. Because of the cooling sensation imparted to the skin, the amount of the cooling composition added to toiletries will usually be in the range 0.1 to 10% by weight based on the total composition. Medicaments will normally feature an amount of the cooling composition of from 0.01 to 2.0% by weight. Tobacco preparations may contain as little as 0.1 mg. of the composition.

In addition to the cooling composition described herein, these products can contain other additives according to use. For example, additives permitted by Food Sanitation Law can be added to food and drink according to necessity. Useful additives include saccharides, sweeteners, inorganic salts, emulsifiers, acidifiers, flavorings, colors, antioxidants, raising agents, thickeners, vegetable oils, milk, and other dairy products. In some detail, bakery products can comprise wheat flour (base), butter, a raising agent, e.g., baking powder, an emulsifier, e.g., a sucrose fatty acid ester, saccharides, e.g., sugar, inorganic salts, and flavorings. Chocolate can comprise cacao mass (base) cacao butter, saccharides, e.g., sugar, milk, and an emulsifier. Emulsified dressings can comprise salad oil, water, vinegar, sugar, thickening polysaccharides, and sweeteners. Chewing gum can comprise a gum base, saccharides, such as sugar, glucose and starch syrup, and flavors. Candy can comprise saccharides, acidifiers, e.g., citric acid, sweeteners, flavorings, and colors. Orange fruit drinks can comprise orange juice, sweeteners, e.g., isomerized sugars, acidifiers, e.g., citric acid, and antioxidants, e.g., vitamin C. Fruit milk drinks can comprise fruit juice, dairy products such as milk and powdered skim milk, saccharides, e.g., sugar, stabilizers, e.g., carboxymethyl cellulose, acidifiers, e.g., citric acid, and flavorings, e.g., a pineapple flavor.

In particular embodiments, the cooling compositions are used in chewing gums. Some chewing gum formulations are described in, for instance, U.S. Patents 6,627,233, 6,685,916 and 6,696,044, herein incorporated by reference. Additives which can be used in the preparations include inorganic salts, inorganic oxides, organic salts, thickeners, wetting agents, emulsifiers, surface active agents, humectants, alcohols, color additives, flavorings, and, if desired, medical ingredients such as crude drugs, hemostatics, circulation stimulants, anti-inflammatory agents, astringents, antibacterial and/or antifungal agents, and bactericides. In particular embodiments, toothpaste can comprise abrasives, such calcium phosphate, as calcium carbonate, aluminum hydroxide, silica, and calcium pyrophosphate; wetting agents, such as glycerin, sorbitol, and propylene glycol; tackifiers, such as carboxymethyl cellulose, carrageenan, and hydroxyethyl cellulose; surface active agents, such as sodium laurylsulfate, N-acylglutaminates, and sucrose fatty acid esters; sweeteners, such as saccharin sodium, stevioside, and xylitol; and medicinal components, such as vitamin E, azulene, aluminum chlorohydroxy allanthoinate, dextranase, hinokitiol, lysozyme chloride, and chlorhexidine.

### Chewing Gum Compositions

In particular embodiments, the cooling compositions are used in chewing gums and confectionaries. Compositions of chewing gum and confectionaries are well known in the art and described in depth in, for instance, U.S. Patents 6,685,916, 6,627,233, 6,685,916 and 6,696,044, the disclosures of which are incorporated by reference and some of which is summarized herein.

Chewing gum compositions typically include one or more of gum bases, flavoring agent and bulk sweeteners. The term "confectioneries" as used herein includes, but is not limited to: nougats, candies, panning goods, gel confections, fondants, lozenges, hard boiled candies, mints, troches, pastilles, microcapsules, and fast-dissolving solid forms including freeze dried forms (cakes, wafers, thin films, and tablets) and fast dissolving solid forms including compressed tablets. The term "fast dissolving solid form" as used herein means that the solid dosage form dissolves in less than about 60 seconds, preferably less than about 15 seconds, more preferably less than about 5 seconds, in the oral cavity. Lozenges include discoid shaped solids comprising a therapeutic agent in a flavored base. The base may be a hard sugar candy, glycerinated gelatin, or a combination of sugar with sufficient mucilage to give it form. Lozenge compositions (compressed tablet type) typically include one or more fillers (compressible sugar), flavoring agents and lubricants.

The chewing gum compositions may be coated or uncoated and be in the form of slabs, sticks, pellets, balls, compressed tablets, and the like. The composition of the different forms of the chewing gum compositions will be similar but may vary with regard to the ratio of the ingredients. For example, coated gum compositions may contain a lower percentage of softeners. Pellets and balls have a small chewing gum core, which can then be coated with either a sugar solution or a sugarless solution to create a hard shell. Slabs and sticks are usually formulated to be softer in texture than the chewing gum core. In order to overcome any detrimental softening effect the surfactant active may have on the gum base, it is preferred to formulate a slab or stick gum having a firmer texture (i.e. with less softener than is typically employed). Compressed tablets are formed from compressible mixtures.

The cooling compositions may be used in either regular chewing gum, pressed gum or bubble gum, a subset of "chewing gums." Center filled gum is another common chewing gum form. The gum portion has a similar composition and mode of manufacture to that described above. However, the center fill is typically an aqueous solution or gel, which can be injected into the center of the gum during processing. The cooling agents and compositions could optionally be incorporated together or singly into the center fill during manufacture of the fill or into the chewing gum. The center fill gum may also be optionally coated and may be prepared in various forms such as in the form of a lollipop.

It is preferred to use a coated gum wherein the cooling compositions described herein are in at least one of the core and the coating.

The chewing gum composition includes gum base and most of the other typical chewing gum composition components such as sweeteners, softeners, flavoring agents and the like. The chewing gum composition may contain a reduced amount of softening agents such as lecithin or glycerin or may eliminate softeners. In addition, the chewing gum composition may contain a larger or smaller amount of sugar alcohols than conventional chewing gum compositions to facilitate delivery.

In accordance with one aspect of the chewing gum composition, the cooling compositions are added during the manufacture of the chewing gum composition, that is, with the sweeteners, flavoring agents and the like.

In a further aspect, the gum base generally comprises elastomers, elastomer plasticizers, waxes, fats, oils, emulsifiers, fillers, texturizers and may include a desirable combination of the heating and cooling agents or warming composition. Elastomers constitute from about 5% to 95% by weight of the base, preferably 10% to 70% by weight and most preferably 15% to 45% by weight. Examples of elastomers include synthetic elastomers such as polyisobutylene, polybutylene, isobutylene-isoprene co-polymers, styrenebutadiene co-polymers, polyvinylacetate and the like. Elastomers may also include natural elastomers such as natural rubber as well as natural gums such as jelutong, lechi caspi, perillo, massaranduba balata, chicle, gutta hang kang or mixtures thereof. Other elastomers are known to those of ordinary skill in the art.

Elastomer plasticizers modify the firmness of the finished gum when used in the gum base. Elastomer plasticizers are typically present in an amount of up to about 75% by weight of the gum base, preferably from about 5% to 45% by weight and more preferably from about 10% to 30% by weight. Examples of elastomer plasticizers include natural rosin esters such as glycerol ester of partially hydrogenated rosin, glycerol ester of tall oil rosin, pentaerythritol esters of partially hydrogenated rosin, methyl and partially hydrogenated methyl esters of rosin, and the like. Synthetic elastomer plasticizers such as terpene resins may also be employed in gum base composition.

Waxes include synthetic and naturally occurring waxes such as polyethylene, bees wax, carnauba and the like. Petroleum waxes such paraffin may also be used. The waxes may be present in the amount of up to about 30% by weight of the gum base. Waxes aid in the curing of the finished gum and help improve the release of flavor and may extend the shelf life of the product.

Fillers modify the texture of the gum base and aid processing. Examples of such fillers include magnesium and aluminum silicates, clay, alumina, talc, titanium oxide, cellulose polymers, and the like. Fillers are typically present in an amount of from 1% to 60% by weight.

Examples of softeners used in the gum base include hydrogenated and partially hydrogenated vegetable oils, cocoa butter, glycerol monostearate, glycerol triacetate, di- and triglycerides, fatty acids such as stearic acid, palmitic acid, oleic acid, linoleic acid, linolenic acid and the like.

The gum base constitutes between 5% and 95% by weight of the chewing gum composition, more typically 10% to 50% by weight, and most typically from about 25% to 35% by weight of the chewing gum. A higher amount of gum base is preferred.

Other ingredients used in chewing gum compositions include sweeteners, both natural and artificial and both sugar and sugarless. Sweeteners are typically present in the chewing gum compositions in amounts of from about 20% to 80% by weight, preferably from about 30% to 60% by weight. Sugarless sweeteners include, but are not limited sugar alcohols such as sorbitol, mannitol, xylitol, hydrogenated starch hydrolysates, maltitol and the like may also be present. High intensity sweeteners such as sucralose, aspartame, neotame, salts of acesulfame, and the like are typically present up to about 1.0% by weight.

Flavoring agents, which can vary over a wide range, may be selected in amounts from about 0.1% to 10.0% by weight, preferably from about 0.5% to 5.0% by weight. Flavoring agents for use in chewing gum compositions are well known and include citrus oils, peppermint oil, spearmint oil, oil of wintergreen, menthol, cinnamon, ginger and the like.

Softeners may be present to modify the texture of the chewing gum composition. As in typical gum compositions, softeners in the compositions are typically present in amounts of from about 0.5% to 10% by weight based on the total weight of the chewing gum composition.

Other materials, which may be present in the gum composition include antioxidants (e.g. butylated hydroxyanisole, butylated hydroxytoluene, beta-carotenes, tocopherols), colorants, flavoring agents and the like.

Coating techniques for applying a coating for a chewing gum composition such as pan and spray coating are well known. Preferred is coating with solutions adapted to build a hard candy layer. Both sugar and sugar alcohols may be used for this purpose together with high intensity sweeteners, colorants, flavoring agents, binders and other conventional additives. When the combination of stain removing agents is provided in the coating of a chewing gum composition, a solution of the stain removing agents is preferably, alternately applied with the flavoring agent.

The sweetener may be present in an amount of from about 30% to 80% by weight of the coating syrup. A binder such as magnesium stearate may be added to the coating syrup in an amount of from about 1% to 15% by weight of the coating syrup to enhance or promote adhesion. Optionally, minor amounts of conventional additives may also be present. The sweeteners suitable for use in the coating syrup comprise sugarless sweeteners such as the polyhydric alcohols, e.g., xylitol, sorbitol, mannitol, and mixtures, thereof; as well as maltitol, isomaltitol, hydrogenated starch hydrolysates, and hydrogenated glucose syrups. Mono, di- and polysaccharide may also be included. For example, sugars such as sucrose, fructose, glucose, galactose and maltose may also be employed as a sweetener. Other sweeteners suitable for use in the coating syrup include, but are not limited to free saccharin acid, water soluble salts of saccharin, cyclamate salts, palatinit dihydrochalcones, glycyrrhizin, L-aspartyl-L-phenylalanine methyl ester, amino acid based sweeteners, talin, steviosides, dihydrochalcone compounds, acesulfame salts and mixtures thereof.

Other ingredients may be added in minor amounts to the coating syrup and include moisture absorbing compounds, anti-adherent compounds, dispersing agents and film forming agents. The moisture absorbing compounds suitable for use in the coating syrups include mannitol or dicalcium phosphate. Examples of useful anti-adherent compounds, which may also function as filler, include talc, magnesium trisilicate and calcium carbonate. These ingredients may be employed in amounts of about 0.5% to 5% by weight of the syrup. Examples of dispersing agents, which may be employed in the coating syrup, include titanium dioxide, talc or other anti-adherent compounds as set forth above.

The coating syrup can be heated and a portion thereof deposited on the cores. Usually a single deposition of the coating syrup is not sufficient to provide the desired amount or thickness of coating and it usually will be necessary to apply second, third or more coats of the coating syrup in order to build up the weight and thickness of the coating to desired levels with layers allowed to dry in-between coats.

In some embodiments of the chewing gum composition, the cooling compositions can be added to the coating. They are preferably applied subsequent to the syrup coating. Further details regarding the preparation of chewing gum compositions can be found in Skuse's Complete Confectioner (13th Edition) (1957) including pp. 41-71, 133-144, and 255-262; and Sugar Confectionery Manufacture (2nd Edition) (1995), E. B. Jackson, Editor, pp. 258-286, the content of which is incorporated herein by reference.

### Confectionary Compositions

The present description also encompasses confectionery products containing a composition of individual agents that in total impart a physiological effect that can be substantially cooling. Confectionery compositions include compressed tablets such as mints, hard boiled candies, nougats, gels, center fill confections, fondants, panning goods and other compositions falling within the generally accepted definition of confectionery compositions.

Confectionery compositions in the form of pressed tablets such as mints may generally be made by combining finely sifted sugar or sugar substitute, flavoring agent (e.g. peppermint flavor), bulking agent such as gum arabic, and an optional coloring agent. The flavoring agent and the bulking agent are combined and then gradually the sugar or sugar substitute are added along with a coloring agent, if needed.

The product then can be granulated by passing through a sieve of desired mesh size (e.g. 12 mesh) and then dried at typically 55° C to 60° C. The resulting powder can be fed into a tableting machine fitted with a large size punch and the resulting pellets are broken into granules and then pressed.

High boiled candies typically contain sugar or sugar substitute, glucose, water, flavoring agent and optional coloring agent. The sugar can be dissolved in the water and glucose can be then added. The mixture can be brought to a boil. The resulting liquid to which may previously have been added a coloring agent can be poured onto an oiled slab and cooled. The flavoring agent then can be added and kneaded into the cooled mass. The resulting mixture then can be fed to a drop roller assembly known in the art to form the final hard candy shape.

A nougat composition typically includes two principal ingredients, a high boiled candy and a frappe. By way of example, egg albumen or substitute thereof can be combined with water and whisked to form a light foam. Sugar and glucose are added to water and boiled typically at about 130°C to 140° C. and the resulting boiled product can be poured into a mixing machine and beat until creamy. The beaten albumen and flavoring agent are combined with the creamy product and the combination can be thereafter thoroughly mixed.

Further details regarding the preparation of confectionery compositions can be found in Skuse's Complete Confectioner (13th Edition) (1957) including pp. 41-71, 133-144, and 255-262; and Sugar Confectionery Manufacture (2nd Edition) (1995), E. B. Jackson, Editor, pp. 129-168, 169-188, 189-216, 218-234, and 236-258, the content of which is incorporated herein by reference.

### Quantitative Descriptive Analysis

Many sensory test methods such as descriptive analysis depend upon the judgments of panelists about the perceived strength of individual sensory characteristics in a product. These perceived intensity estimates are often given as scale numbers or choices on a rating scale. Judgments are often made relative to some frame of reference, which is sometimes explicit in the training of the panel. However, human judgments tend to shift as a function of the immediate context, and the set of products evaluated within a sensory test session can skew the ratings of an individual judge. There can be a stabilizing effect of training and reference standards in reducing such context effects and the resistance of different scaling methods to contextual shifting.

One text covering all the basic techniques of sensory testing is Sensory Evaluation of Food: Principles & Practices, by Harry T. Lawless and Hildegarde Heymann, the disclosure of which is herein incorporated by reference. Statistics used in sensory evaluation are demonstrated as integrated applications in the context of appropriate sensory methods and are also presented as stand-alone material in appendices. Statistical applications are tailored to common analyses encountered to sensory work, together with instructions on how tests should be conducted. One exemplary application of the principles of quantitative descriptive analysis with respect to milk products is provided by Chapman et al., J. Dairy Science 84:12-20 (2001).

A panel of respondents may be assembled for sensory evaluation. Attribute terms for evaluation of samples are selected. Normally, ballot development and respondent training is carried out initially. Descriptive terms are developed for major sensory attribute categories. Exemplary attribute qualities include aroma, flavor, texture, aftertaste, sweetness, etc. Attributes are quantified with an intensity scale of from, e.g. 0 to 10; where 0 indicates that the attribute is not detected and 10 indicates the attribute is extremely strong. Overall quality rating may be measured with a scale of from, e.g. 1 to 10 where less than 6 is considered "poor," 6 to 7 is "fair," and 8 to 10 is "good."

Physical reference standards are determined by a panel consensus so that proper descriptive language may be developed. Panelists may be trained in evaluating certain samples until a consensus is attained.

Overall quality ratings and quantified intensity ratings may be analyzed with such programs as Minitab ver. 12 or SAS ver. 6.11. Descriptive statistical measures may be calculated for all attributes. Analysis of variance may be performed on each attribute using a randomized block design for balanced data with panelists as repeated measures as described by Ott, "Analysis of variance for some standard experimental designs," pp. 844-856 in An Introduction to Statistical Methods and Data Analysis. Wadsworth Publishing. Belmont, CA. Where F-test indicates a significant difference between treatment means, Tukey paired comparisons and orthogonal comparisons may be used to determine where the means are different. Significance of differences may be defined as P less than 0.05. Principal components analysis (PCA) may be applied with the factor analysis described by Lawless and Heymann, 1998, pp. 606-608 in Sensory Evaluation of Food: Principles and Practices. Chapman & Hall, New York, 1998. PCA may be applied to the attributes. Attributes may be omitted if the values are consistently low indicating that the attribute is not often present, if the attribute has a high standard deviation or if the attribute is highly correlated to another attribute. Kaiser's criterion may be applied (eigen value greater than 1) to determine the number of final factors from the initial ones as described by Massart et al., "Principal components and factor analysis," pp. 339-369 in Chemometrics: A Textbook. Elsevier, Amsterdam, 1988. To facilitate the interpretation of results, the factors may be orthogonally rotated leading to uncorrelated factors following the Varimax method described by Massart et al., *supra.*

The overall quality ratings (dependent variables) may be modeled as a function of the Varimax rotated PC scores for the products (independent variables). Models may be constructed using ordinary least squares (OLS), principal components regression (PCR), and partial least squares regression (PLS) routines in SCAN for Windows Release 1.1. PCR and PLS models may be calculated with, for instance, one to four components. In each case, the best fit equations (those with the highest R²) and those with the best predictive ability (lowest residual predictive sum of squares, or residual PRESS) are obtained.

Further, respondents can score each product at three time points; one minute, fifteen minutes, and thirty minutes for overall liking and intensity of four attributes: flavor, sweetness, cooling/warming and texture. A fifteen minute rest period can be provided between each product allowing for palate cleansing. The tests can be performed generally according to the criterion set forth above. Such statistical processes as set forth above may be used to analyze the data collected.

The present cooling compositions and methods will now be illustrated in greater detail with reference to Examples in view of Comparative Examples, but it should be understood that the methods and compositions are not limited thereto. Unless otherwise noted, all the percents are by weight.

In some embodiments, cooling compositions can be formulated to contain a menthyl glutarate ester in the following amounts expressed as weight percent. Additionally, the cooling compositions can be coupled with a flavor composition as indicated. WS-3 and WS-23 are shown together to indicate that they can be used interchangeably.

**Table 1**

| | | Cooling Compositions |
|---|---|---|
| 1. | Menthyl lactate | 5 - 60% |
| | Menthol | 15 - 85% |
| | WS-3 or WS-23 | 5 - 50% |
| 2. | Menthyl glutarate | 5 - 60% |
| | Menthol | 15 - 85% |
| | WS-3 or WS-23 | 5 - 50% |
| 3. | Menthyl succinate | 5 - 60% |
| | Menthol | 15 - 85% |
| | WS-3 or WS-23 | 5 - 50% |

**Table 2**

| | | Chewing Gum Compositions |
|---|---|---|
| 1. | Gum Base | 20 - 50% |
| | Sugar | 35 - 80% |
| | Glucose Syrup | 8 - 25% |
| | Sweetener | 0.01 - 1.5% |
| | Lecithin | 0.2 - 1.2% |
| | Glycerin | 1 - 8% |
| | Color | 0.01 - 0.05% |
| | Peppermint Flavor | 1.2 - 2.5% |
| | Cooling Composition | 0.2 - 1.2% |
| 2. | Gum Base | 20 - 50% |
| | Sugar | 35 - 80% |
| | Glucose Syrup | 8 - 25% |
| | Sweetener | 0.01 - 1.5% |
| | Lecithin | 0.2 - 1.2% |
| | Glycerin | 1 - 8% |
| | Color | 0.01 - 0.05% |
| | Wintergreen Flavor | 0.9 - 2.3% |
| | Peppermint Flavor | 0.5 - 1.5% |
| | Cooling Composition | 0.2 - 1.2% |
| 3. | Gum Base | 20 - 50% |
| | Sugar | 35 - 80% |
| | Glucose Syrup | 8 - 25% |
| | Sweetener | 0.01 - 1.5% |
| | Lecithin | 0.2 - 1.2% |
| | Glycerin | 1 - 8% |
| | Color | 0.01 - 0.05% |
| | Spearmint Flavor | 1.2 - 2.5% |
| | Cooling Composition | 0.2 - 1.2% |
| 4. | Gum Base | 20 - 50% |
| | Sugar | 35 - 80% |
| | Glucose Syrup | 8 - 25% |
| | Sweetener | 0.01 - 1.5% |
| | Lecithin | 0.2 - 1.2% |
| | Glycerin | 1 - 8% |
| | Color | 0.01 - 0.05% |
| | Cinnamon Flavor | 1.5 - 2.5% |
| | Cooling Composition | 0.2 - 1.2% |
| 5. | Gum Base | 20 - 50% |
| | Sugar | 35 - 80% |
| | Glucose Syrup | 8 - 25% |
| | Sweetener | 0.01 - 1.5% |
| | Lecithin | 0.2 - 1.2% |
| | Glycerin | 1 - 8% |
| | Color | 0.01 - 0.05% |
| | Fruit Flavor | 1.2 - 2.5% |
| | Cooling Composition | 0.2 - 1.2% |
| 6. | Gum Base | 20 - 50% |
| | Sorbitol Powder | 35 - 80% |
| | Sweetener | 0.01 - 1.5% |
| | Lecithin | 0.2 - 1.2% |
| | Glycerin | 3- 15% |
| | Color | 0.01 - 0.05% |
| | Peppermint Flavor | 1.2 - 2.5% |
| | Cooling Composition | 0.2 - 1.2% |

### Chewing Gum and Confectionary Compositions Providing A Physiological Cooling Sensation

In some embodiments, chewing gum and confectionaries will be prepared having some of the components provided below in amounts within those amounts recommended by the United States government as not exceeding the amounts set forth below. The chewing gums and confectionary compositions will demonstrate an ability to impart a physiological cooling sensation.

**TABLE 3**

| GRAS # | Compound names | FEMA recomm. levels in GUM | FEMA recomm. levels in HARD CANDY | FEMA recomm. levels in SOFT CANDY |
|---|---|---|---|---|
| 2665 | Menthol | 1,100 | 400 | 400 |
| 2667 | Menthone | 8.7 | 71 | 71 |
| 2668 | Menthyl acetate | 5.2 | 26 | 26 |
| 2962 | Isopulegol | NA | 23 | 23 |
| 3455 | WS-3 | 1,200 | 10 | 10 |
| 3460 | Isomenthone | 600 | 60 | 60 |
| 3748 | Menthyl Lactate | 800 | NA | NA |
| 3784 | 3-1-menthoxypropane-1,2-diol | 4,000 | 500 | 500 |
| 3804 | WS-23 | 3,000 | 50 | NA |
| 3805 | 1-menthol ethylene glycol carbonate | 20,000 | 2,000 | NA |
| 3806 | 1-menthol-1and2-propylene glycol carbonate | 10,000 | 3,000 | NA |
| 3807 | 1-menthone1,2 glycerol ketal | 800 | NA | 80 |
| 3808 | d,l-menthone 1,2 glycerol ketal | 800 | NA | 80 |
| 3810 | Mono-menthyl succinate (physcool) | 3,750 | 600 | 600 |
| 3992 | d,l menthol propyleneglycol carbonate | 20,000 | 2,000 | 2,000 |
| 4006 | L-monomenthyl glutarate | 4,000 | 700 | 600 |

From the foregoing description, various modifications and changes in the compositions and methods will occur to those skilled in the art. All such modifications coming within the scope of the appended claims are intended to be included therein.

All publications, including but not limited to patents and patent applications, cited in this specification are herein incorporated by reference as if each individual publication were specifically and individually fully set forth.

### Annex to Description

1. A cooling composition comprising a menthyl ester and a second cooling agent.
2. A composition according to item 1, further comprising menthol.
3. A cooling composition comprising a menthyl ester, menthol, and a third cooling agent.
4. A composition according to item 1, wherein the second cooling agent is selected from the group consisting of N-ethyl-p-menthane-3-carboxamide (WS-3) and trimethyl isopropyl butanamide (WS-23).
5. A composition according to item 1, wherein the menthyl ester is present in an amount of at least 5 weight percentage of the composition.
6. A composition according to item 1, wherein the menthyl ester is present in an amount of at least 10 weight percentage of the composition.
7. A composition according to item 1, wherein the menthyl ester is present in an amount of at least 20 weight percentage of the composition.
8. A composition according to item 1, wherein the menthyl ester is present in an amount of at least 30 weight percentage of the composition.
9. A composition according to item 1, wherein the menthyl ester is present in an amount of at least 40 weight percentage of the composition.
10. A composition according to item 1, wherein the menthyl ester is present in an amount of 5 to 60 weight percentage of the composition.
11. A composition according to item 1, wherein the menthyl ester is present in an amount of 15 to 50 weight percentage of the composition.
12. A composition according to item 1, wherein the menthyl ester is monomenthyl glutarate ester.
13. A composition according to item 1, wherein the menthyl ester is selected from the group consisting of monomenthyl succinate, dimenthyl succinate, monomenthyl [alpha],[alpha]-dimethyl succinate, monomenthyl 2-methylmaleatementhyl glutarate (FEMA 4006), monomenthyl glutarate ester, dimenthyl glutarate ester, and a menthyl half acid ester derivative.
14. A chewing gum having a cooling composition comprising a menthyl ester and a second cooling agent.
15. A chewing gum according to item 14, wherein the cooling composition further comprises menthol.
16. A chewing gum according to item 14, wherein the second cooling agent is selected from the group consisting of N-ethyl-p-menthane-3-carboxamide (WS-3) and trimethyl isopropyl butanamide (WS-23).
17. A chewing gum according to item 14, wherein the menthyl ester is present in an amount of at least 5 weight percentage of the cooling composition.
18. A chewing gum according to item 14, wherein the menthyl ester is present in an amount of at least 10 weight percentage of the cooling composition.
19. A chewing gum according to item 14, wherein the menthyl ester is present in an amount of at least 20 weight percentage of the cooling composition.
20. A chewing gum according to item 14, wherein the menthyl ester is present in an amount of at least 30 weight percentage of the cooling composition.
21. A chewing gum according to item 14, wherein the menthyl ester is present in an amount of at least 40 weight percentage of the cooling composition.
22. A chewing gum according to item 14, wherein the menthyl ester is present in an amount of 0.005 to 1.2 weight percentage of the chewing gum.
23. A chewing gum according to item 14, wherein the menthyl ester is present in an amount of 15 to 50 weight percentage of the cooling composition.
24. A chewing gum according to item 14, wherein the menthyl ester is mononienthyl glutarate ester.
25. A confection having a cooling composition comprising a menthyl glutarate ester and a second cooling agent.
26. A confection according to item 25, wherein the cooling composition further comprises menthol.
27. A confection according to item 25, wherein the second cooling agent is selected from the group consisting of N-ethyl-p-menthane-3-carboxamide (WS-3) and trimethyl isopropyl butanamide (WS-23).
28. A confection according to item 25, wherein the menthyl ester is present in an amount of at least 5 weight percentage of the cooling composition.
29. A confection according to item 25, wherein the menthyl ester is present in an amount of at least 10 weight percentage of the cooling composition.
30. A confection according to item 25, wherein the menthyl ester is present in an amount of at least 20 weight percentage of the cooling composition.
31. A confection according to item 25, wherein the menthyl ester is present in an amount of at least 30 weight percentage of the cooling composition.
32. A confection according to item 25, wherein the menthyl ester is present in an amount of at least 40 weight percentage of the cooling composition.
33. A confection according to item 25, wherein the menthyl ester is present in an amount of 0.0005 to 1.2 weight percentage of the confection.
34. A confection according to item 25, wherein the menthyl ester is present in an amount of 15 to 50 weight percentage of the cooling composition.
35. A confection according to item 25, wherein the menthyl ester is monomenthyl glutarate ester.
36. A method for delivering a prolonged physiological cooling sensation to the skin or a mucous membrane comprising administering a composition according to item 1.
37. A method for delivering a sensation substantially similar to that delivered by N-ethyl-p-menthane-3-carboxamide (WS-3) alone comprising administering a composition according to item 1 wherein the amount of N-ethyl-p-menthane-3-carboxamide (WS-3) present in the composition is reduced relative to the amount of N-ethyl-p-menthane-3- carboxamide (WS-3) alone required to provide the substantially similar sensation.
38. A method for delivering a sensation substantially similar to that delivered by trimethyl isopropyl butanamide (WS-23) alone comprising administering a composition according to item 1 wherein the amount of trimethyl isopropyl butanamide (WS-23) present in the composition is reduced relative to the amount of trimethyl isopropyl butanamide (WS- 23) alone required to provide the substantially similar sensation.

## Claims

1. A cooling composition comprising
5 to 60 weight percent of a menthyl glutarate ester;
5 to 50 weight percent of N-2,3-trimethyl-2-isopropyl butanamide (WS-23); and
15 to 85 weight percent of menthol.

2. A chewing gum comprising a cooling composition according to claim 1.

3. The chewing gum of claim 2, wherein the cooling composition is present in an amount of 0.2 to 1.2 weight percent of the chewing gum.

4. A confection comprising a cooling composition according to claim 1.

5. A method for delivering a prolonged physiological cooling sensation to the skin or a mucous membrane comprising administering a composition according to claim 1.

6. A method for delivering a sensation substantially similar to that delivered by N-2,3-trimethyl-2-isopropyl butanamide alone comprising administering a composition according to claim 1, wherein the amount of N-2,3-trimethyl-2-isopropyl butanamide present in the composition is reduced relative to the amount of N-2,3-trimethyl-2-isopropyl butanamide alone required to provide the substantially similar sensation.
